# EUROPÄISCHE PATENTSCHRIFT

(11) **EP 3 299 052 B1**
(45) Veröffentlichungstag und Bekanntmachung des Hinweises auf die Patenterteilung: **11.09.2019**
(21) Anmeldenummer: 17194892.0
(22) Anmeldetag: 25.11.2010
(51) Int. Cl.: A61M 5/315

(54) **DOSIERVORRICHTUNG FÜR EINE INJEKTIONSVORRICHTUNG**
METERING DEVICE FOR AN INJECTION DEVICE
DISPOSITIF DE DOSAGE POUR UN DISPOSITIF D'INJECTION

(43) Veröffentlichungstag der Anmeldung: 28.03.2018
(62) Teilanmeldung aus: 10787352.3
(73) Patentinhaber: Tecpharma Licensing AG, 3401 Burgdorf (CH)
(72) Erfinder: Moser, Ulrich, 3412 Heimiswil (CH); Hirschel, Jürg, 3007 Bern (CH); Meier, Stefan, 3250 Lyss (CH); Schrul, Christian, 3400 Burgdorf (CH)

(56) Entgegenhaltungen:
- WO-A1-2007/030957
- US-A- 5 092 842
- US-A- 5 383 865
- US-A1- 2005 033 224

## Beschreibung

Die vorliegende Erfindung bezieht sich auf eine Dosiervorrichtung für eine Injektionsvorrichtung gemäss dem Oberbegriff des Anspruchs 1.

Des Weiteren bezieht sich die Erfindung auf ein Verfahren zum Einstellen und Abgeben einer Dosis aus einer Injektionsvorrichtung nach dem Oberbegriff des Anspruchs 13.

Aus der WO02/092153A2 ist eine Injektionsvorrichtung mit einer Dosiervorrichtung bekannt.

Aus der US 5,383,865 ist eine Injektionsvorrichtung mit einer Dosiervorrichtung gemäß dem Oberbegriff des Anspruchs 1 mit einem Kupplungsmechanismus bekannt.

Ferner ist aus der US 5,092,842 ist eine Injektionsvorrichtung mit einer Dosiervorrichtung mit einem Rückdrehsicherungsmechanismus bekannt.

Der vorliegenden Erfindung liegt somit die Aufgabe zugrunde, eine alternative Dosiervorrichtung für eine Injektionsvorrichtung und ein alternatives Verfahren zum Einstellen und Abgeben einer Dosis aus einer Injektionsvorrichtung bereit zu stellen.

Diese Aufgabe wird durch den Gegenstand mit den Merkmalen des Anspruchs 1 und dem Verfahren gemäss Anspruch 15 gelöst.

Vorteilhafte Ausgestaltungen der Erfindung sind in den abhängigen Ansprüchen angegeben.

Die Erfindung betrifft eine Dosiervorrichtung für eine Injektionsvorrichtung mit einem Betätigungselement zum Einstellen und Abgeben einer Dosis, wobei das Betätigungselement zum Einstellen einer aus der Injektionsvorrichtung abzugebenden Dosis aus der Dosiervorrichtung bewegt, insbesondere herausgeschraubt werden kann. Das Betätigungselement kann sich dabei in die proximale Richtung bewegen. Zum Abgeben einer Dosis kann das Betätigungselement wieder vollständig in die Dosiervorrichtung bewegt, insbesondere hinein geschraubt und in die distale Richtung bewegt werden. Beispielsweise kann das Betätigungselement in einem Gehäuse der Injektionsvorrichtung oder in einem Gehäuse der Dosiervorrichtung, insbesondere in einem Gewindeeingriff geführt werden.

Eine Kolbenstange zum Erzeugen einer Vorschubbewegung zum Ausschütten einer Dosis ist in der Injektionsvorrichtung, insbesondere der Dosiervorrichtung beweglich gelagert. Die Kolbenstange kann an ihrem distalen Ende einen Anschlag, insbesondere einen Flansch aufweisen, welcher auf einen in einer Karpule enthaltenen Kolben stossen kann, wobei der Flansch den Kolben axial in die Karpule hinein schieben kann, um ein in der Karpule enthaltendes Produkt, insbesondere eine Flüssigkeit oder ein Medikament aus der Injektionsvorrichtung abzugeben. Die Kolbenstange kann ein Gewinde aufweisen und in einer Gewindeführung der Injektionsvorrichtung, insbesondere der Dosiervorrichtung geführt werden.

Die Injektionsvorrichtung, insbesondere die Dosiervorrichtung weist ferner eine Kolbenstangenhülse auf, wobei in der Kolbenstangenhülse die Kolbenstange aufgenommen ist. Die Kolbenstange ist mit der Kolbenstangenhülse drehfest verbindbar. Die Kolbenstange ist über eine Kupplungshülse mit der Kolbenstangenhülse drehfest verbindbar. Die Kupplungshülse kann in Eingriff mit der Kolbenstange und der Kolbenstangehülse sein, um eine drehfeste Verbindung zu bilden. Die Kolbenstange kann relativ zu der Kolbenstangenhülse gedreht werden, wenn die Kupplungshülse ausser Eingriff mit der Kolbenstange und der Kolbenstangenhülse ist.

Ein Kupplungselement, welches das Betätigungselement und die Kolbenstangenhülse bei einer Ausschüttbewegung des Betätigungselements derart koppelt, dass eine Ausschüttbewegung des Betätigungselements über die mit der Kolbenstange verbundenen Kolbenstangenhülse auf die Kolbenstange übertragen werden kann. Bei einer Kupplung während der Ausschüttbewegung sind das Betätigungselement und die Kolbenstangenhülse drehfest miteinander verbindbar. Die Kupplung zwischen dem Betätigungselement und dem Kupplungselement kann des Weiteren derart gebildet sein, dass während einer Einstellbewegung oder einer Dosiskorrektur das Betätigungselement von der Kolbenstangenhülse entkoppelt sein kann. Während der Ausschüttbewegung wird mittels des Kupplungselements das Betätigungselement mit der Kolbenstangenhülse verdrehsicher gekoppelt, wobei die Drehbewegung aufgrund der drehfesten Verbindung zwischen der Kolbenstangenhülse und der Kolbenstange auf die Kolbenstange übertragen werden kann. Das Kupplungselement weist ein Koppelglied und das Betätigungselement weist ein Gegenkoppelglied auf, welche durch eine radiale Verschiebung des Kupplungselements relativ zu dem Betätigungselements zueinander eingekoppelt oder geöffnet werden können. Vorzugsweise wird durch die relative Drehbewegung ein taktiles, visuelles und/oder akustisches Signal erzeugt, sodass der Benutzer erkennen kann, dass er eine Einstellbewegung oder eine Dosiskorrektur tätigt. Das Kupplungselement kann durch eine radiale Verschiebung des Kupplungselements, insbesondere mindestens eines Teils des Kupplungselements relativ zu dem Betätigungselements mit dem Betätigungselements in Eingriff kommen. Eine kombinierte axiale und radiale Bewegung des Kupplungselements ist auch denkbar.

Die Dosiervorrichtung kann einen Auslöseknopf aufweisen, welcher drehbar an dem Betätigungselement gelagert ist. Der Dosierknopf kann derart ausgebildet sein, dass er bei einer Vorschubbewegung in die distale Richtung auf das Kupplungselement wirkt und eine axiale und/oder radiale Verschiebung oder Kraftübertragung des Kupplungselements relativ zu dem Betätigungsglied ermöglicht.

In der Dosiervorrichtung ist eine Rückdrehsicherung in Form eines Rückdrehsicherungselements für die Kolbenstangenhülse vorgesehen, um eine Drehung der Kolbenstangenhülse in eine Richtung zu ermöglichen und in Gegenrichtung zu sperren. Somit kann sich die Kolbenstangenhülse nur in eine Richtung zur Dosiervorrichtung drehen. In die Gegenrichtung kann eine Drehung der Kolbenstangenhülse relativ zu der Dosiervorrichtung durch das Rückdrehsicherungselement verhindert werden. Die Kolbenstangenhülse kann mit dem Kupplungselement verdrehsicher verbunden sein. Die Rückdrehsicherung kann derart ausgebildet sein, dass bei der Einstellung einer Dosis die Kolbenstangenhülse und das Kupplungselement relativ zu der Dosiervorrichtung nicht gedreht werden können und bei Ausschüttung einer Dosis, die Kolbenstangenhülse und das Kupplungselement relativ zu der Dosiervorrichtung gedreht werden können. Das Kupplungselement kann während der Einstellbewegung relativ zu dem Betätigungselement drehbar und axial fest sein, wohingegen während der Ausschüttbewegung aufgrund des Kupplungsmechanismus zwischen dem Betätigungselement und dem Kupplungselement das Kupplungselement relativ zu dem Betätigungselement drehfest und axialfest verbunden sein kann und somit die Ausschüttdrehung auf die Kolbenstangenhülse und die mit der Kolbenstangenhülse drehfest verbundenen Kolbenstange übertragen werden kann. Durch diese Anordnung kann sich ergeben, dass während der Einstellung einer Dosis das Kupplungselement relativ zu der Kolbenstangenhülse eine translatorische Bewegung ausführen kann, wobei das Kupplungselement mit dem Betätigungselement in die distale Richtung bewegt werden kann, und dass das Kupplungselement und die Kolbenstangenhülse sich nicht relativ zu der Dosiervorrichtung drehen können. Während der Ausschüttung einer Dosis kann sich das Kupplungselement relativ zu der Kolbenstangenhülse translatorisch in die proximale Richtung bewegen, wobei das Kupplungselement und die Kolbenstangenhülse sich relativ zu der Dosiervorrichtung drehen können.

Vorzugsweise ist das notwendige Drehmoment für eine Bewegung zwischen dem Rückdrehsicherungsglied und der Kolbenstangenhülse grösser als das notwendige Drehmoment für eine Bewegung zwischen dem Betätigungselement und dem Kupplungselement. Die Bewegung des Betätigungselements bei einer Dosisabgabe und einer Dosiskorrektur ist prinzipiell gleich, wobei bei der Dosiskorrektur der Kopplungsmechanismus zwischen dem Betätigungselement und dem Kupplungselement nicht kraftschlüssig verbunden ist und bei einer Dosisabgabe kraftschlüssig verbunden sein kann. Dadurch kann bewirkt werden, dass während einer Dosiskorrektur die Kolbenstangenhülse relativ zu der Dosiervorrichtung drehfest ist und keine Ausschüttung des Produkts durch die Kolbenstange stattfindet. Wohingegen während einer Dosisabgabe die Kolbenstangenhülse relativ zu der Dosiervorrichtung drehbar ist, um durch die Kolbenstange eine Dosis abzugeben.

In einer bevorzugten Ausführungsform ist die Rückdrehsicherung in Form eines Ratschenmechanismus in der Dosiervorrichtung vorgesehen, mit welcher die Kolbenstangenhülse und die Kolbenstange relativ zu der Dosiervorrichtung in nur eine Richtung, vorzugsweise in die Drehrichtung drehbar ist, in der die Kolbenstange in Ausschüttrichtung bewegbar ist. In die Gegenrichtung, vorzugsweise in die entgegen gesetzte Drehrichtung kann die Kolbenstangenhülse durch den Ratschenmechanismus gesperrt werden. Damit ist beispielsweise nur ein in die distale Richtung gerichtetes Schrauben der in der Dosierungsvorrichtung gewindegeführten Gewindestange möglich. Das Rückdrehsicherungselement kann derart in Eingriff mit der Kolbenstangenhülse sein, dass das Rückdrehsicherungselement eine Drehung der Kobenstangenhülse in eine Richtung, vorzugsweise in die Drehrichtung, ermöglicht und in die Gegenrichtung, vorzugsweise in die entgegen gesetzte Drehrichtung, sperrt. Das Rückdrehsicherungselement kann mit der Kolbenstangenhülse gekoppelt sein. Das Rückdrehsicherungselement und die Kolbenstangenhülse können mittels einer Verzahnung oder Verrastung in Eingriff sein. Die Kolbenstangenhülse kann ein Zahn oder Vorsprung aufweisen und an dem Rückdrehsicherungselement kann ein korrespondierender Zahn oder Vorsprung vorgesehen sein, welcher den Zahn der Kolbenstangenhülse aufnehmen kann. An den entsprechenden Bestandteilen der Dosiervorrichtung können auch mehrere Zähne oder Vorsprünge beziehungsweise mehrere korrespondierenden Zähne oder Vorsprünge gebildet sein. Die Flanken der Verzahnung oder Verrastung können derart ausgebildet sein, dass die Kolbenstangenhülse nur unidirektional relativ zu der Dosiervorrichtung gedreht werden kann. Vorzugsweise kann das Rückdrehsicherungselement zumindest teilweise spannbar oder elastische deformierbar ausgebildet sein. Alternativ kann das Rückdrehsicherungselement vollständig spannbar oder elastisch deformierbar ausgebildet sein. Das Rückdrehsicherungselement kann federnd ausgebildet sein oder ein Federelement aufweisen. Beispielsweise kann an dem Rückdrehsicherungselement ein Federarm oder mehrere Federarme vorgesehen sein oder als Federarm ausgebildet sein. Der Federarm des Rückdrehsicherungselements kann derart mit dem Zahn oder Anschlag der Kolbenstangenhülse zusammenwirken, dass eine unidirektionale Drehung des Rückdrehsicherungselements relativ zu der Kolbenstangenhülse vollführbar ist. Das Rückdrehsicherungselement kann mit einem Gehäuse der Dosiervorrichtung dreh- und axialfest verbunden sein. Das Rückdrehsicherungselement kann federnd in die Kolbenstangenhülse eingreifen, um eine Drehung der Kolbenstangenhülse in eine Richtung zu ermöglichen und in die Gegenrichtung zu sperren, wobei bei der Drehung der Kolbenstangenhülse relativ zu dem Rückdrehsicherungselement ein visuelles, taktiles und/oder akustisches Signal, vorzugsweise ein Klick-Geräusch erzeugt werden kann. Die Rückdrehsicherung kann auch auf andere Weise ausgebildet sein. Beispielsweise kann an dem Rückdrehsicherungselement beziehungsweise an der Kolbenstangenhülse ein oder mehrere Vorsprünge oder Stege vorgesehen sein oder als ein oder mehrere Vorsprünge oder Stege ausgebildet sein, welche beispielsweise in eine oder mehrere Nuten oder Ausnehmungen der Kolbenstangenhülse beziehungsweise des Rückdrehsicherungselements eingreifen können. In einer anderen Ausführungsform kann die Kolbenstangenhülse zumindest teilweise spannbar oder elastisch deformierbar ausgebildet sein. Die Kolbenstangenhülse kann federnd ausgebildet sein oder ein Federelement aufweisen und mit dem Rückdrehsicherungselement derart zusammenwirken, dass die Kolbenstangenhülse relativ zu der Rückdrehsicherungselement in eine Drehrichtung drehbar ist. Die relative Drehung kann mit einem visuellen, taktilen und/oder akustischen Signal, vorzugsweise ein Klick-Geräusch angezeigt werden.

Die Dosiervorrichtung kann eine Gewindeführung für die ein Gewinde aufweisende Kolbenstange umfassen. Die Dosiervorrichtung kann eine Hülse aufweisen, welche axialfest und vorzugsweise drehfest mit der Dosiervorrichtung verbindbar ist und die Gewindeführung bildet. Bei einer Ausschüttbewegung kann die Kolbenstangenhülse die Drehbewegung an die Kupplungshülse übertragen, wobei die Kupplungshülse drehfest mit der Kolbenstange verbunden sein kann, sodass die Drehbewegung der Kolbenstangenhülse über die Kupplungshülse auf die Kolbenstange übertragbar ist. Ein beispielsweise an der Kolbenstange angeordnetes Aussengewinde kann beispielsweise in einen Gewindeeingriff mit einer an dem Gehäuse axialfest und vorzugsweise drehfest angeordneten Gewindehülse geführt werden, wobei durch die Führung der Gewindehülse die Drehbewegung der Kolbenstange zu einer axialen Bewegung der Kolbenstange führen kann. Die Kolbenstange mit dem an der Kolbenstange drehbar angeordneten Flansch kann sich in die distale Richtung bewegen, wobei die Ausschüttkraft von dem Flansch der Kolbenstange auf den Kolben übertragen werden kann, sodass der Kolben relativ zu der Karpule verschoben wird und das Produkt aus der Karpule ausgeschüttet werden kann.

Vorzugsweise umfasst das Betätigungselement ein Stoppglied, welches mit einem Gegenstoppglied des Gehäuses der Dosiervorrichtung zusammenwirken kann, sodass die Einstellung einer maximalen Dosis begrenzt werden kann. Das Betätigungselement kann in einer Gewindeführung des Gehäuses der Dosiervorrichtung geführt werden. Bei Erreichen einer maximal einstellbaren Dosis aus der Injektionsvorrichtung abzugebenden Dosis kann beispielsweise ein Anschlag des Betätigungselements gegen einen Gegenanschlag des Gehäuses stossen. Der Anschlag und der Gegenanschlag können beispielsweise als an einem Ende des Gewindes des Betätigungselements, vorzugsweise an einem distalen Ende und als an einem Ende des Gewindes des Gehäuses, vorzugsweise an einem proximalen Ende beispielsweise als Axial- und/oder Radialanschlag und als Axial- und/oder Radialgegenanschlag vorgesehen sein. Vorzugsweise können an einem anderen Ende des Gewindes des Betätigungselements, vorzugsweise an einem proximalen Ende und an einem anderen Ende des Gewindes des Gehäuses, vorzugsweise an einem proximalen Ende ein weiteres Stoppglied und ein weiteres Gegenstoppglied beziehungsweise ein weiterer Anschlag und ein weiterer Gegenanschlag vorhanden sein, welche die Ausschüttbewegung des Betätigungselements gegenüber dem Gehäuse begrenzen. Die Begrenzung kann durch einen Axial- und/oder Radialanschlag erfolgen. Die Anschläge können auch als Schnapparme ausgebildet sein.

Bei der Ausschüttbewegung bewegt sich die Kolbenstange relativ zu der Dosiervorrichtung in die distale Richtung. An dem proximalen Ende der Kolbenstange kann ein weiteres Stoppglied vorgesehen sein, welches mit einem an der Gewindehülse angebrachten Gegenstoppglied derart koppeln kann, dass die axiale Bewegung der Kolbenstange relativ zu der Dosiervorrichtung gestoppt werden kann. Die axiale Bewegung der Kolbenstange ist nicht mehr möglich, wenn beispielsweise ein Anschlag der Kolbenstange auf einen Gegenanschlag der Gewindehülse anliegt. Die Anschläge können als Axial- und/oder Radialanschlag und als Axial- und/oder Radialgegenanschlag ausgebildet sein. Eine weitere axiale Bewegung der Kolbenstange kann somit verhindert werden, insbesondere wenn kein Produkt mehr in der Karpule enthalten ist. Eine Dosismenge, welche bereits ausgeschüttet wurde beziehungsweise, welche noch nicht ausgeschüttet wurde, kann an einer Dosisanzeige des Betätigungselements abgelesen werden, wobei das Betätigungselement die Dosisanzeige bilden kann oder die Dosisanzeige ein separates Teil, beispielsweise eine Dosisanzeigehülse und mit dem Betätigungselement axialfest und vorzugsweise drehfest verbunden oder verbindbar ist.

In einer bevorzugten Ausführungsform ist die Dosiervorrichtung derart ausgebildet, dass ein Karpulenwechsel vorgenommen werden kann, bei welchem eine leere Karpule, bei der das Produkt ausgeschüttet wurde, mit einer mit dem Produkt gefüllten Karpule ausgewechselt werden kann. Die Karpule kann in einem Karpulenhalter aufgenommen sein oder als Karpulenhalter ausgebildet sein, wobei dann der Karpulenwechsel mit dem Karpulenhalter oder mit dem die Karpule bildenden Karpulenhalter vollzogen werden kann.

Der Karpulenhalter kann mit der Dosiervorrichtung lösbar verbunden sein. Die Verbindung kann ein Bajonett-, ein Schnapp- oder ein Drehverschluss sein. Wenn das Produkt vollständig oder zumindest teilweise aus der Injektionsvorrichtung ausgeschüttet ist, kann die Kolbenstange in die distale Richtung aus der Dosiervorrichtung heraus ragen. Der Karpulenhalter kann von der Dosiervorrichtung abgenommen werden, wobei die Kolbenstange in einer distalen Position innerhalb der Dosiervorrichtung gelagert sein kann. Die Kupplungshülse kann ausser Eingriff mit der Kolbenstangenhülse gelangen, sodass die Kolbenstange relativ zu der Kolbenstangenhülse drehbar ist. Bei Druckausübung auf den Flansch der Kolbenstange in proximale Richtung kann die Kolbenstange zurück in die Dosiervorrichtung verschoben werden. Die Kolbenstange kann aufgrund der Gewindeführung mit der Hülse, insbesondere der Gewindehülse, welche axialfest und vorzugsweise drehfest mit der Dosiervorrichtung verbindbar ist, in die Dosiervorrichtung in eine proximale Position hinein geschraubt werden.

Die Kupplungshülse kann über einen Kupplungsmechanismus in und ausser Eingriff mit der Kolbenstangenhülse gebracht werden. Der Kupplungsmechanismus kann mindestens ein Teil, insbesondere zwei Teile umfassen, welche derart relativ zu der Kupplungshülse und insbesondere zueinander angeordnet sind, dass die Kupplungshülse in und ausser Eingriff mit der Kolbenstangenhülse gebracht werden kann. Der Kupplungsmechanismus kann einen Kupplungsring und einen Kupplungshalter umfassen, welche in einem Führungseingriff sein können. Bei Drehung des Kupplungshalters kann der Kupplungsring aufgrund des Führungseingriffs, insbesondere durch eine Nockenführung zwischen dem Kupplungshalter und dem Kupplungsring, wobei der Kupplungshalter eine Führung und der Kupplungsring eine Nocke aufweisen kann, zwangsgeführt werden. Die Führungsbahn kann derart ausgebildet sein, dass sich der Kupplungsring relativ zu der Gewindehülse axial und/oder drehbar bewegen kann. In einer bevorzugten Ausführungsform ist die Führungsbahn in dem Kupplungshalter derart ausgebildet, dass bei einer Drehung des Kupplungshalters, der Kupplungsring von distal nach proximal verschoben werden kann. Der Kupplungsring kann axial fest und drehbar mit der Kupplungshülse verbunden sein.

Beim Einsetzen des Karpulenhalters kann die Kolbenstange zurück in die Dosiervorrichtung in die proximale Position geschoben werden, bis das distale Ende des Karpulenhalters durch eine Führung an der Gewindehülse in die Dosiervorrichtung geschoben werden und den Kupplungshalter aufnehmen und eine drehfeste Verbindung mit den Kupplungshalter herstellen kann. Bei einer Drehung des Karpulenhalters mit dem Kupplungshalters relativ zu der Gewindehülse kann die Kupplungshülse über den Kupplungshalter und den Kupplungsring in Eingriff mit der Kolbenstangenhülse gebracht werden. Zwischen der Gewindehülse und dem Karpulenhalter kann eine Verbindung hergestellt werden, insbesondere ein Bajonettverbindung.

Beim Abnehmen des Karpulenhalters von der Dosiervorrichtung kann die Verbindung, insbesondere die Bajonettverbindung zwischen dem Karpulenhalter und der Gewindehülse gelöst werden. Aufgrund einer drehfesten Verbindung zwischen dem Karpulenhalter und dem Kupplungshalter und aufgrund der Führung zwischen dem Kupplungshalter und des Kupplungsrings kann die Kupplungshülse ausser Eingriff mit der Kolbenstangenhülse gelangen.

Die Erfindung wird anhand von Ausführungsbeispielen beschrieben. Die dabei offenbarten Merkmale bilden je einzeln und in Kombination auch mit den vorher genannten Ausführungsformen den Gegenstand der Erfindung, welcher durch die Patentansprüche definiert ist, vorteilhaft weiter.

Es zeigen:
- Figur 1: eine Explositionsansicht einer beispielhaften Dosiervorrichtung
- Figur 2: eine Injektionsvorrichtung mit einem Karpulenhalter und einer beispielhaften Dosiervorrichtung in einem Auslieferungszustand
- Figur 3a: eine Injektionsvorrichtung mit einem Karpulenhalter und einer beispielhaften Dosiervorrichtung in Längsschnittansicht in einem zusammengesetzten Zustand vor Einstellung einer Dosis
- Figur 3b: die in Figur 3a gezeigte Injektionsvorrichtung in Aussenansicht
- Figur 4a: die in Figur 3a gezeigte Injektionsvorrichtung in Längsschnittansicht nach Einstellung einer Dosis in einem aufgezogenen Zustand
- Figur 4b: die in Figur 4a gezeigt Injektionsvorrichtung in Aussenansicht
- Figur 5: die in Figur 3a gezeigt Injektionsvorrichtung in Queransicht bei Einstellung einer maximalen Dosis, wobei die Queransicht der in Fig. 4a eingezeichneten Querschnittlinie X-X entspricht
- Figur 6: die in Figur 3a gezeigt Injektionsvorrichtung in Perspektivansicht nach Ausschüttung einer Dosis
- Figur 7: die in Figur 3a gezeigte Injektionsvorrichtung in Queransicht nach Ausschüttung einer letzten Dosis, wobei die Queransicht der in Fig. 12a eingezeichneten Querschnittlinie Y-Y entspricht
- Figur 8: eine Kupplung nach der Erfindung zwischen Betätigungselement und Kupplungselement
- Figur 9: eine beispielhafte Kupplung zwischen Rückdrehsicherungselement und Kolbenstangenhülse
- Figur 10a: eine Längsansicht einer beispielhaften Dosiervorrichtung nach Ausschüttung der letzten Dosis, wobei die Dosiervorrichtung von dem Karpulenhalter losgelöst ist
- Figur 10b: die in Figur 10a gezeigt Dosiervorrichtung in Perspektivansicht
- Figur 11a: eine Längsansicht einer beispielhaften Dosiervorrichtung beim Einsetzen eines Karpulenhalters
- Figur 11b: die in Figur 11b gezeigte Dosiervorrichtung in Perspektivansicht

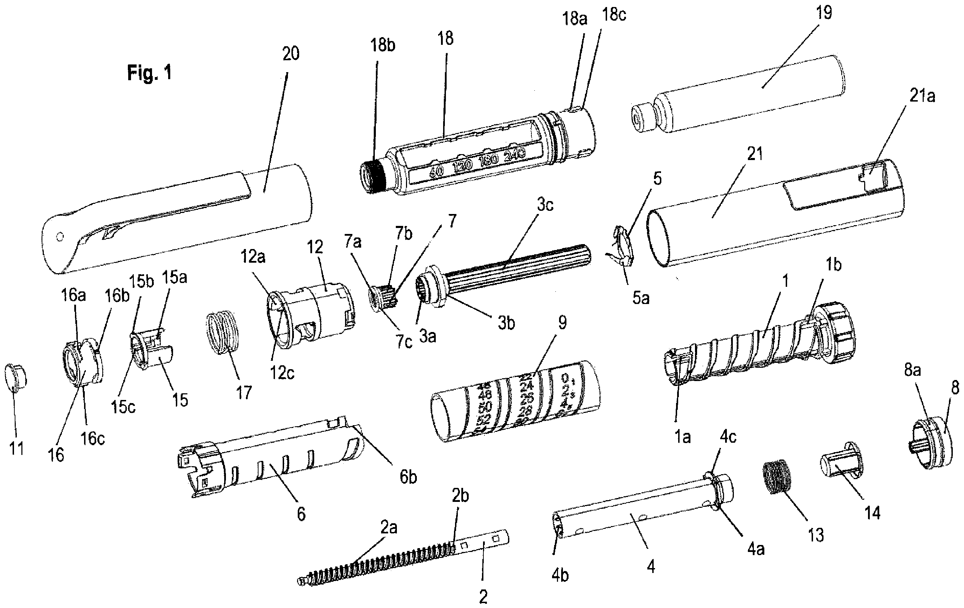

Figur 1 zeigt eine beispielhafte Dosiervorrichtung für eine Injektionsvorrichtung in Explosionsansicht. Die erfindungsgemässe Dosiervorrichtung soll im Kontext der beispielhaften Dosiervorrichtung gelesen werden. Die beispielhafte Dosiervorrichtung umfasst ein Betätigungselement (1) zum Einstellen und Abgeben einer Dosis. Das Betätigungselement (1) weist ein Aussengewinde auf, welches in ein Innengewinde eines Gehäuses (6) der Dosiervorrichtung geführt wird. Zum Einstellen einer Dosis wird das Betätigungselement (1) aus dem Gehäuse (6) geschraubt, während das Betätigungselement (1) zur Abgabe einer Dosis in das Gehäuse (6) zurück geschraubt wird. An dem Betätigungselement (1) ist eine Anzeigehülse (9) axial- und drehfest angeordnet, um die aufgezogene, abgegebene oder noch abzugebende Dosis durch ein Anzeigefenster (21a) einer Aussenhülse (21), welche das Gehäuse (6) koaxial umhüllt, anzuzeigen. In der Dosiervorrichtung ist eine Kolbenstange (2) mit einem Aussengewinde zum Erzeugen einer Vorschubbewegung zum Ausschütten einer Dosis beweglich gelagert. Am distalen Ende der Kolbenstange ist ein Flansch (11) drehbar angeordnet. Bei der Vorschubbewegung der Kolbenstange (2) stösst der Flansch (11) an einen Kolben, der in einer Karpule (19) beweglich aufgenommen ist, um ein Produkt aus der Injektionsvorrichtung abzugeben. Die Karpule (19) ist in einem Karpulenhalter (18) aufgenommen, wobei der Karpulenhalter (18) mit der Karpule (19) von einer Schutzkappe (20) abnehmbar koaxial umgeben ist. Das Aussengewinde der Kolbenstange (2) ist in einem Gewindeeingriff mit einem Innengewinde einer Gewindehülse (12). Die Gewindehülse (12) ist axial- und drehfest über die Aussenhülse (21) mit dem Gehäuse (6) verbunden. Die Gewindehülse (12) dient dazu, eine Drehbewegung der Kolbenstange (2) in eine kombinierte Dreh- und Axialbewegung der Kolbenstange (2) zu transformieren. Eine Kolbenstangenhülse (3) umgibt die Kolbenstange (2) koaxial. Eine Kupplungshülse (7) verbindet die Kolbenstange (2) lösbar mit der Kolbenstangenhülse (3), wobei die Kupplungshülse (3) eine drehfeste Verbindung mit der Kolbenstange (2) eingeht. Die Kupplungshülse (7) weist an einer Innenmantelfläche einen nach innen ragenden Steg (7a) auf, welcher eine in Längsrichtung erstreckende Nut (2a) der Kolbenstange (2) eingreift. An einer Aussenmantelfläche der Kupplungshülse (7) sind in Längsrichtung erstreckende Rippen (7b) angeordnet, die in Eingriff mit Rillen (3a) der Kolbenstangenhülse (3) gelangen können. Die Rillen (3a) sind an einer Innenmantelfläche der Kolbenstangenhülse (3) an deren distalem Ende vorgesehen und erstrecken sich in Längsrichtung. Ein Kupplungselement (4) kann das Betätigungselement (1) und die Kolbenstangenhülse (3) derart koppeln, dass eine Ausschüttbewegung des Betätigungselements über die mit der Kolbenstange (2) verbundene Kolbenstangenhülse (3) auf die Kolbenstange (2) übertragen werden kann. An einer Innenmantelfläche des Kupplungselements (4) sind in Längsrichtung erstreckende Rippen (4b) angeordnet, welche in Längsrichtung erstreckende an einer Aussenmantelfläche der Kolbenstangenhülse (3) angeordnete korrespondierende Rippen (3c) eingreifen, um eine drehfeste Verbindung zwischen dem Kupplungselement (4) und der Kolbenstangenhülse (3) herzustellen. Das Kupplungselement (4) kann sich aber aufgrund der Rippenverbindung axial relativ zu der Kolbenstangenhülse (3) bewegen. Das Kupplungselement (4) weist an einer Aussenmantelfläche ein Koppelglied (4a), welches in an einer Innenmantelfläche angeordnetes Gegenkoppelglied (1c) des Betätigungselements (1) eingreifen kann.

Ein Drehknopf (8) ist drehbar in dem Betätigungselement (1) durch eine Schnappverbindung gelagert. Eine Dosierfeder (13) ist innerhalb des Drehknopfs (8) angeordnet und stützt sich am distalen Ende an dem Kupplungselement (4) und am proximalen Ende an einem Federhalter (14) ab, welcher mittels Punktauflage in dem Drehknopf (8) gehalten wird, wobei der Federhalter (14) gegen eine distale Stirnseite des Dosierknopfs (8) anstösst. Die Dosiervorrichtung umfasst ferner ein Rückdrehsicherungselement (5), das zumindest teilweise, vorzugsweise vollständig, federnd ausgebildet und einen nach distaler Richtung ragenden Federarm (5a) aufweist, wobei das Rückdrehsicherungselement (5) mit dem Gehäuse (6) dreh- und axialfest verbunden ist. Der Federarm (5a) des Rückdrehsicherungselements (5) greift in eine Verzahnung (3b) der Kolbenstangenhülse (3) ein, welche an einer Aussenmantelfläche der Kolbenstangenhülse (3) vorgesehen ist. Der Federarm (5a) und die Verzahnung (3b) sind derart ausgebildet, dass die Kolbenstangenhülse (3) nur unidirektional relativ zu dem Federarm (5a) gedreht werden kann. Eine relative Drehbewegung zwischen der Kolbenstangenhülse (3) und dem Federarm (5a) kann ein Klick-Geräusch erzeugen. In der Gewindehülse (12) sind die Kupplungshülse (7), ein Kupplungsring (15), ein Kupplungshalter (16) und eine Kupplungsfeder (17) gelagert. Die Kupplungshülse (7) weist am distalen Ende einen an einer Aussenmantelfläche angeordneten nach aussen ragenden Ring (7c) auf. Der Kupplungsring (15) weist an der Innenmantelfläche in Längsrichtung erstreckende Stege auf, die derart relativ zueinander angeordnet sind, dass die Stege an der Innenmantelfläche des Kupplungrings (15) eine Ringnut (15a) bilden. Der Ring (7c) der Kupplungshülse (7) ist in der Ringnut (15a) des Kupplungsrings (15) drehbar aber axial fest aufgenommen. Am distalen Ende des Kupplungsrings (15) ist eine an einer Mantelaussenfläche angebrachte nach aussen ragende Führungsnocke (15b) angeordnet, welche in einer Führungsbahn (16a) des Kupplungshalters (16) geführt werden kann. Die Führungsbahn (16a) ist in der Mantelfläche des Kupplungshalters (16) als Durchbruch ausgebildet. Des Weiteren ist die Führungsbahn (16a) des Kupplungshalters (16) derart ausgebildet, dass der Führungsnocken (15b) des Kupplungsrings (15) bei einer Drehung des Kupplungsrings (15) relativ zu der Gewindehülse (12) von distal nach proximal geführt werden kann. Die Kupplungsfeder (17) ist mit ihrem proximalen Ende an der Gewindehülse (12) und mit ihrem distalen Ende an dem Kupplungshalter (16) abgestützt und beaufschlagt den Kupplungshalter in distale Richtung mit einer Federkraft. Der Kupplungshalter (16) ist axial verschieblich und drehbar relativ zu der Gewindehülse (12) angeordnet, wohingegen der Kupplungsring (15) relativ zu der Gewindehülse (12) axial beweglich aber drehfest ist. Der Kupplungshalter (16) weist einen an einer Aussenmantelfläche vorgesehenen nach aussen ragenden Vorsprung (16b) auf, welcher in einer an einer Innenmantelfläche der Gewindehülse angeordneten axialen Nut (nicht ersichtlich) mit einer Ausnehmung geführt werden kann, um den Kupplungshalter (16) mit einem eingesetzten Karpulenhalter (18) in einer Rastposition zu fixieren. An einer Innenmantelfläche des Kupplungsrings (15) ist ein in Längsrichtung nach innen ragender Steg (15c) angebracht, welcher in einer an einer Innenmantelfläche angeordneten Längsnut (12c) der Gewindehülse (12) aufgenommen ist. Aufgrund der Führungskulisse zwischen dem Kupplungshalter (16) und dem Kupplungsring (15) kann durch eine Drehung des Kupplungshalters relativ zu der Gewindehülse (12) der Kupplungsring (15) eine axiale Bewegung zu der Gewindehülse (12) vollführen.

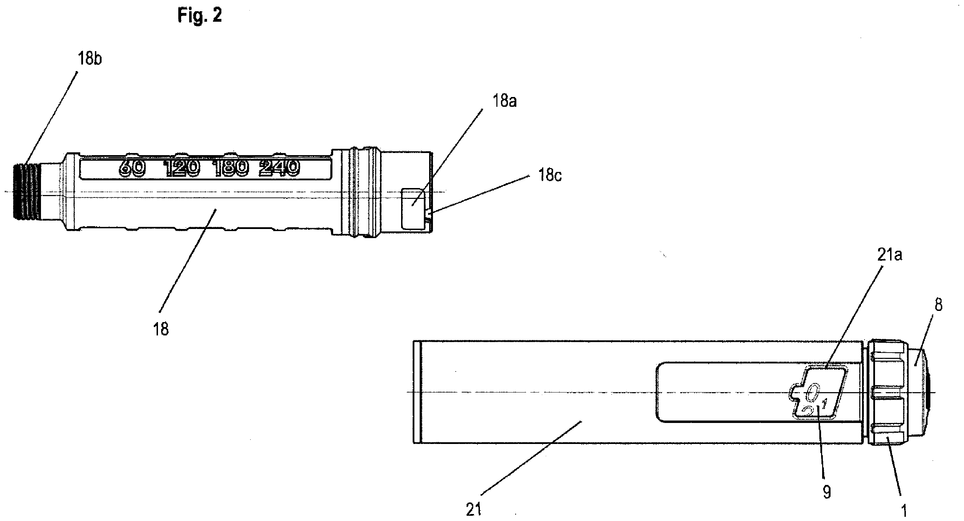

Figur 2 zeigt die beispielhafte Dosiervorrichtung und den Karpulenhalter (18) mit der darin aufgenommenen Karpule (19) in einem Auslieferungszustand. Das distale Ende des Karpulenhalters (18) weist ein Gewinde (18b) auf, um eine Nadeleinheit mit der Karpule (19) zu verbinden, sodass eine fluide Verbindung zwischen der Karpule und einer Nadel entstehen kann. Am proximalen Ende des Karpulenhalters (18) ist an einer Karpulenhalteraussenmantelfläche eine nach aussen ragende Verschlussnocke (18a) vorgesehen, welche in Eingriff mit einer an einer Gewindehülseinnenmantelfläche angeordneten korrespondierenden Verschlussführung (12a) gebracht werden kann, um einen Bajonettverschluss zu bilden. Die Anzeigehülse (9) ist dreh- und axialfest mit dem Betätigungselement (1) verbunden. Das Betätigungselement (1) kann zum Einstellen einer Dosis aus der Dosiervorrichtung gedreht und zum Abgeben einer Dosis in die Dosiervorrichtung gedreht werden. Die Dosis auf der Anzeigehülse (9) kann durch das Anzeigefenster (21a) in der Aussenhülse (21) abgelesen werden.

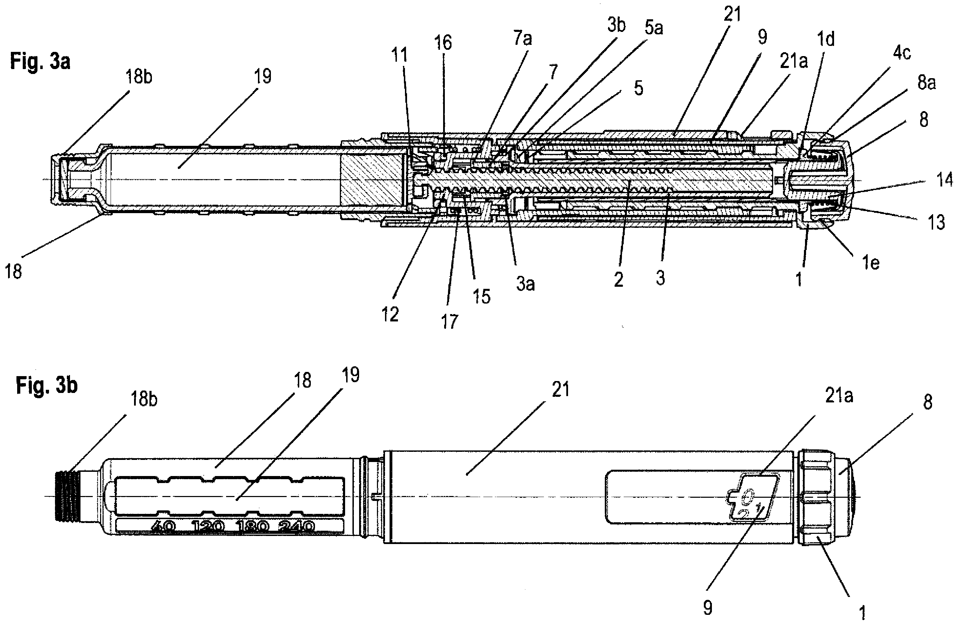

Figur 3a zeigt die beispielhafte Dosiervorrichtung mit dem eingesetzten Karpulenhalter (18) vor Einstellung einer Dosis. Das Aussengewinde des Betätigungselements (1) ist mit dem Innengewinde des Gehäuses (6) in Eingriff. Zwischen dem Gehäuse (6) und einer Aussenhülse (21) ist radial ein Ringspalt angeordnet, in welcher die Anzeigehülse (9) einführbar ist. Die Anzeigehülse ist dreh- und axialfest mit einem Betätigungselement (1) verbunden. Das Betätigungselement kann zum Einstellen einer Dosis aus der Dosiervorrichtung gedreht und zum Abgeben einer Dosis in die Dosiervorrichtung gedreht werden. Die Dosis auf der Anzeigehülse (9) kann durch das Anzeigefenster (21a) in der Aussenhülse (21) abgelesen werden. Das Kupplungselement (4) ist hülsenförmig ausgebildet und ist radial zwischen dem Betätigungselement (1) und der Kolbenstangenhülse (3) angeordnet, wobei in der Kolbenstangenhülse (3) die Kolbenstange (2) koaxial aufgenommen ist. Das Kupplungselement (4) ist über eine Rippenverbindung mit der Kolbenstangenhülse drehfest aber axial verschieblich verbunden. Das Kupplungselement (4) weist am proximalen Ende an einer Aussenmantelfläche einen nach aussen ragenden Ring (4c) des Kupplungselements (4) auf, welcher in Kontakt mit einer in proximaler Richtung angeordneter Stirnfläche (1d) der Betätigungshülse (1) ist. Die Dosierfeder (13), welche zwischen dem Ring (4c) des Kupplungselements (4) und dem Federhalter (14) angeordnet ist, beaufschlagt das Kupplungselement (4) mit einer Federkraft in die distale Richtung und bringt den Ring (4c) in Kontakt mit der Stirnfläche (1d) der Betätigungshülse (1). Die Spannkraft der Dosierfeder ist aber so gering, dass zwischen dem Koppelglied (4a) des Kupplungselements (4) und dem Gegenkoppelglied (1c) des Betätigungselements (1) eine formschlüssige aber keine kraftschlüssige Verbindung hergestellt werden kann. Das Betätigungselement (1) kann sich relativ zu dem Kupplungselement (4) drehen. Der Dosierknopf (8) ist über eine Schnappverbindung mit dem Betätigungselement (1) drehbar verbunden, wobei innerhalb des Dosierknopfs (8) der Federhalter (14) mittels Punktauflage in proximale Richtung an den Dosierknopf (8) gelagert ist. Des Weiteren ragt das proximale Ende des Kupplungselements (4) in den Dosierknopf (8) hinein. An dem distalen Ende der Kolbenstangenhülse (3) sind Zähne (3b) vorgesehen, welche in die proximale Richtung ragen und umlaufend an der Aussenmantelfläche der Kolbenstangenhülse (3) angeordnet sind, wobei die Zähne (3b) radial nach aussen gerichtet sind. Das Rückdrehsicherungselement (5) weist einen Federarm (5a) auf, welcher in die Zähne (3b) der Kolbenstangenhülse (3) greifen. Das Rückdrehsicherungselement (5) ist axial- und drehfest mit dem Gehäuse (6) verbunden, wobei das Gehäuse wiederum mit der Aussenhülse (21) axial- und drehfest verbunden ist. Das Rückdrehsicherungselement (5) und die Zähne (3b) der Kolbenstangenhülse (3) sind derart ausgebildet, dass bei einer Einstellbewegung des Betätigungselements (1) eine relative Bewegung zwischen dem Rückdrehsicherungselement (5) und der Kolbenstangenhülse (3) verhinderbar ist.

Figur 3b zeigt die beispielhafte Dosiervorrichtung mit dem eingesetzten Karpulenhalter (18) vor Einstellung einer Dosis in Aussenansicht.

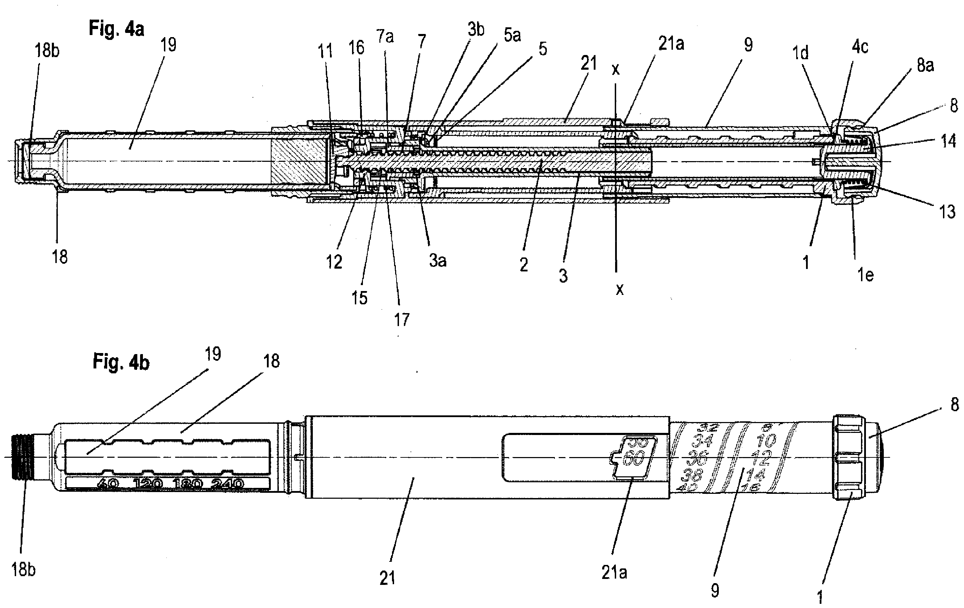

Figur 4a zeigt die beispielhafte Dosiervorrichtung mit dem eingesetzten Karpulenhalter (18) nach Einstellung einer Dosis. Durch Drehung des Betätigungselements (1) schraubt sich diese aufgrund des Gewindeeingriffs mit dem Gehäuse (6) aus der Dosiervorrichtung heraus. Aufgrund der festen Verbindung zwischen dem Betätigungselements (1) und der Anzeigehülse (9) schraubt sich die Anzeigehülse (9) ebenfalls aus der Dosiervorrichtung heraus. Die Federarme (5a) des Rückdrehsicherungselements (5) sind im Eingriff mit den Zähnen (3b). Die Flanken des Federarme (5a) sind derart mit den Flanken der Zähne (3b) in Anschlagkontakt, dass eine relative Drehbewegung zwischen dem Rückdrehsicherungselement (5) und der Kolbenstangenhülse (3) verhindert wird. Aufgrund der Rippenverbindung zwischen der Kolbenstangenhülse (3) und dem Kupplungselement (4), der relativen Drehung zwischen dem Betätigungselement (1) und dem Kupplungselement (4) und der axial festen Verbindung zwischen dem Betätigungselement (1) und dem Kupplungselement (4) wird während der Einstellbewegung das Kupplungselement (4) mit dem Betätigungselement (1) in die proximale Richtung bewegt, wobei sich das Kupplungselement (4) nur axial zu der Dosiervorrichtung bewegt. Die Kolbenstange (2) bewegt sich während der Einstellbewegung nicht, da die Kolbenstange (2) über die Kupplungshülse (7) und die rotationsfeste Kolbenstangenhülse fixiert ist. Für die Dosiskorrektur wird das Betätigungselement (1) zusammen mit der Anzeigehülse (9) in die Dosiervorrichtung zurückgeschraubt. Das Drehmoment zwischen dem Rückdrehsicherungselement (5) und der Kolbenstangenhülse (3) ist grösser als das Drehmoment zwischen dem Betätigungselement (1) und dem Kupplungselement (4). Das Kupplungselement (4) wird durch die Vorspannung der Dosierfeder (13) mit dem Betätigungselement (1) in die Dosiervorrichtung geschoben. Das Kupplungselement (4) wird während der Dosiskorrektur nur durch eine axiale Bewegung in die Dosiervorrichtung zurückgeschoben.

Figur 4b zeigt die beispielhafte Dosiervorrichtung mit dem eingesetzten Karpulenhalter (18) nach Einstellung einer Dosis in Aussenansicht.

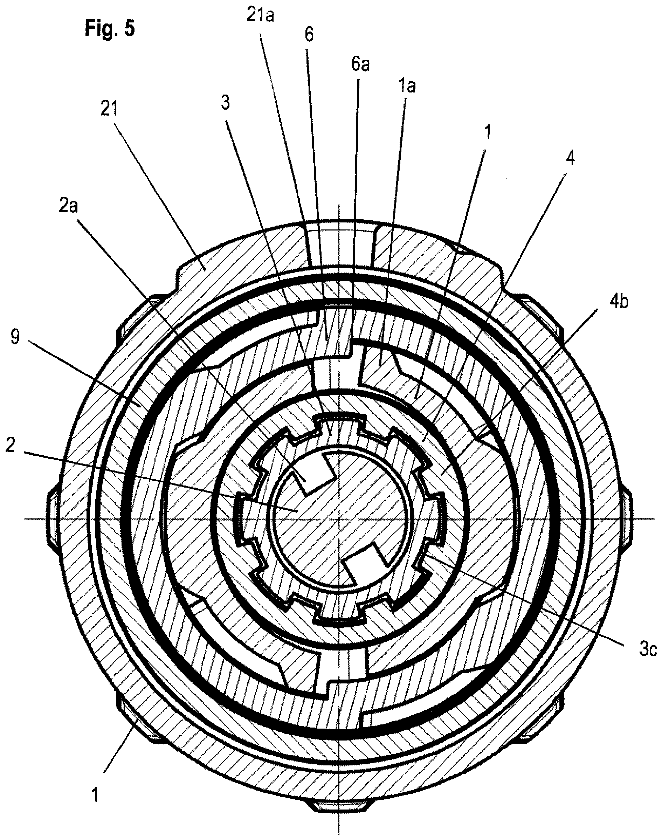

Figur 5 zeigt eine beispielhafte Dosiervorrichtung mit dem eingesetzten Karpulenhalter (18) bei einer Einstellung einer maximalen Dosis. Zum Einstellen einer Dosis wird das Betätigungselement (1) aus dem Gehäuse (6) geschraubt. An einem distalen Ende des Aussengewindes ist eine Anschlagsnocke (1a), insbesondere eine Aufziehnocke (1a) angebracht, welche an eine korrespondierende Gegenanschlagsnocke (6a), insbesondere an eine Aufziehgegenanschlagsnocke (6a), welche an einem proximalen Ende an einer Innenmantelfläche des Gehäuses (6) vorgesehen ist, anschlagbar ist, um die Einstellung einer maximalen Dosis zu beschränken. In Anschlagkontakt kann das Betätigungselement (1) nicht mehr weiter aus dem Gehäuse (6) herausgeschraubt werden.

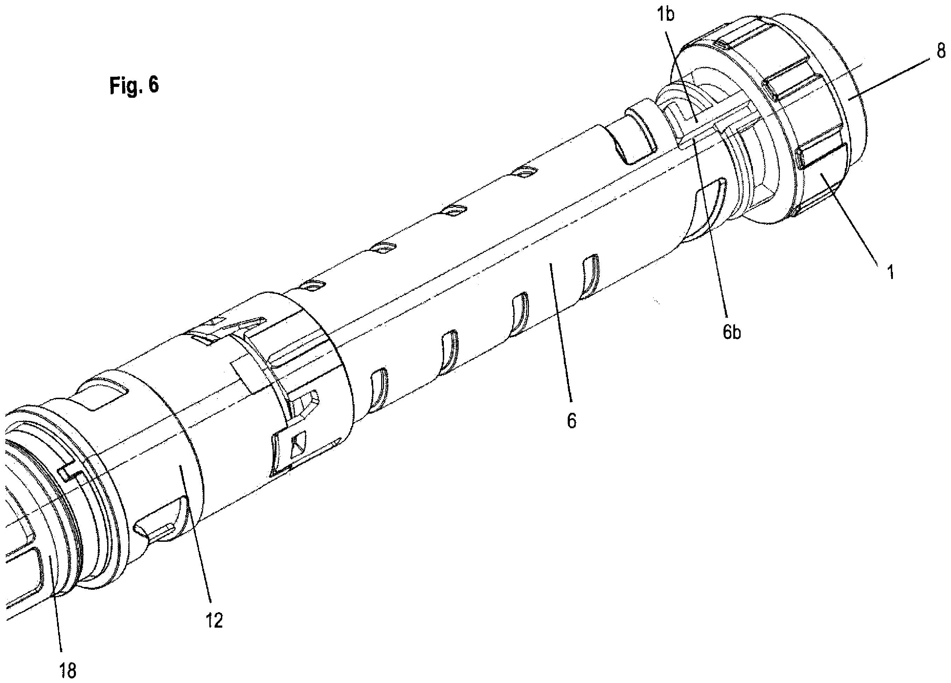

Figur 6 zeigt eine beispielhafte Dosiervorrichtung mit dem eingesetzten Karpulenhalter (18) bei einer Ausschüttung einer Dosis, wobei die Aussenhülse und die Anzeigehülse in dieser Darstellung nicht ersichtlich sind. Zur Abgabe einer Dosis wird das Betätigungselement (1) zur Abgabe einer Dosis in das Gehäuse (6) zurück geschraubt. An dem proximalen Ende des Aussengewindes ist eine weitere Anschlagsnocke (1b), insbesondere eine Ausschüttnocke (1b) vorgesehen, welche mit einer Kante (6b), welche an einem proximalen Ende des Gehäuses (6) angeordnet ist, zusammenwirken kann, wenn eine aufgezogene Dosis vollständig ausgeschüttet ist. In Anschlagkontakt kann das Betätigungselement (1) kein weiteres Produkt aus der Karpule ausschütten.

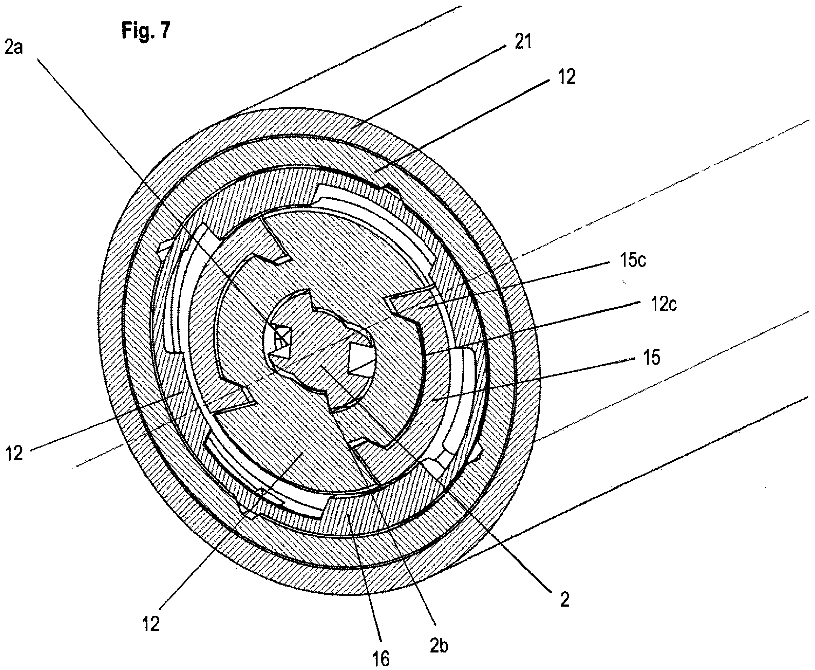

Figur 7 zeigt die beispielhafte Dosiervorrichtung mit dem eingesetzten Karpulenhalter (18) nach Ausschüttung einer letzten Dosis. Die Kolbenstange (2) ist mit dem Aussengewinde in Gewindeeingriff mit der Gewindehülse (12), welche axial- und drehfest über die Aussenhülse (21) mit dem Gehäuse (6) verbunden ist. Die rotierende Kolbenstange (2) bewegt sich aufgrund des Gewindeeingriffs mit der Gewindehülse (12) in distale Richtung. Am proximalen Ende der Kolbenstange (2) ist eine Anschlagnocke (2b), insbesondere ein Stopp der letzten Dosis vorgesehen, welche mit einem Gegenanschlag an der Mantelinnenfläche der Gewindehülse (12) in Anschlagkontakt kommen kann, wenn die Karpule (19) leer ist, beziehungsweise wenn die Kolbenstange in der distalen Position ist.

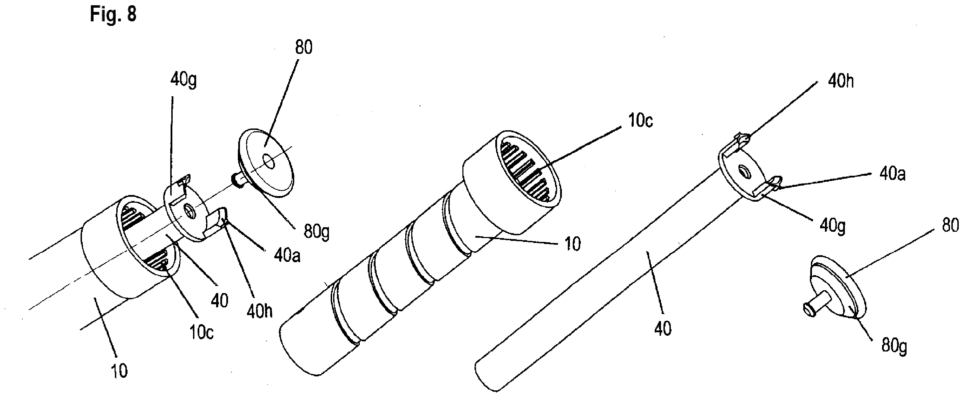

Figur 8 zeigt eine Kupplung der Erfindung zwischen Betätigungselement (10) und Kupplungselement (40). An einer Aussenmantelfläche eines Kupplungselements (40) ist ein Koppelglied als ein radial nach aussen beweglichen Schnapparm (40g) mit einer nach aussen ragenden Nocke (40a) ausgebildet. An einer Innenmantelfläche eines Betätigungselements (10) ist ein Gegenkoppelglied (10c) als nach innen ragende in Längsrichtung erstreckende Rippen (10c) ausgebildet. Ein Dosierknopf (80) ist konusförmig ausgebildet. Während einer Einstellbewegung und einer Dosiskorrektur rattert die Nocke (40a) des Kupplungselements (40) über die Rippen (10c) des Betätigungselements (10), wobei ein Klick-Geräusch erzeugt werden kann. Bei einer Ausschüttbewegung wird der Dosierknopf (80) in das Kupplungselement (40) geschoben, wobei eine schräge Fläche (80g) des Dosierknopfs (80) einen Druck auf eine schräge Fläche (40h) des Kupplungselements (40) ausübt, wobei eine radiale Kraft gegen eine elastische Kraft des Schnapparms (40g) wirkt. Die Nocke (40a) des Schnapparms (40g) des Kupplungselements (40) wird durch die Kraftausübung und durch den konusförmigen Dosierknopf (80) zwischen die Rippen (10c) des Betätigungselements (10) radial nach aussen gedrückt. Die Nocke (40a) des Kupplungselements (40) gerät in Eingriff mit den Rippen (10c) des Betätigungselements (10) und bildet eine drehfeste Verbindung. Nach der Ausschüttungbewegung wird der Dosierknopf (80) durch die elastische Rückstellkraft des Schnapparms (40g) in die Ausgangsposition bewegt. Die Rückstellkraft kann durch ein zusätzliches radial oder axial wirkendes Federelement unterstützt oder erzeugt werden.

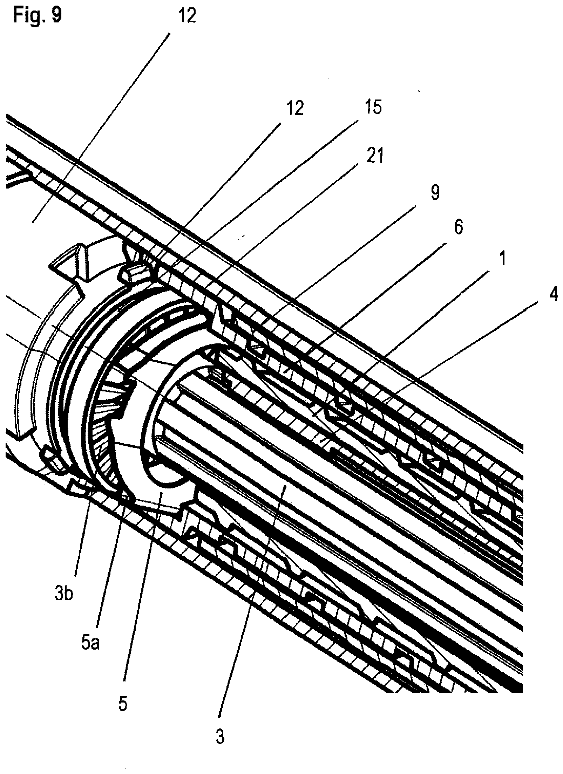

Figur 9 zeigt eine beispielhafte Kupplung zwischen Rückdrehsicherungselement (5) und Kolbenstangenhülse (3). In der Dosiervorrichtung ist eine Rückdrehsicherung vorgesehen. An dem distalen Ende der Kolbenstangenhülse (3) sind Zähne (3b) vorgesehen, welche in die proximale Richtung ragen und umlaufend an der Aussenmantelfläche der Kolbenstangenhülse (3) angeordnet sind. Das Rückdrehsicherungselement (5) weist einen Federarm (5a) auf, welcher in die Zähne (3b) der Kolbenstangenhülse (3) greifen. Das Rückdrehsicherungselement (5) ist axial- und drehfest mit dem Gehäuse (6) verbunden, wobei das Gehäuse wiederum mit der Aussenhülse (21) axial- und drehfest verbunden ist. Das Rückdrehsicherungselement (5) und die Zähne (3b) der Kolbenstangenhülse (3) sind derart ausgebildet, dass die Kolbenstangenhülse (3) nur unidirektional relativ zu dem Rückdrehsicherungselement (5) gedreht werden kann. Der Federarm (5a) des Rückdrehsicherungselements (5) und die Zähne (3b) der Kolbenstangenhülse (3) sind derart ausgebildet, dass bei einer Einstellbewegung und einer Dosiskorrektur des Betätigungselements (1) eine relative Bewegung zwischen dem Rückdrehsicherungselement (5) und der Kolbenstangenhülse (3) verhindert werden kann und bei einer Abgabebewegung des Betätigungselements (1) eine relative Bewegung zwischen dem Rückdrehsicherungselement (5) und der Kolbenstangenhülse (3) zugelassen werden kann. Eine relative Drehbewegung zwischen der Kolbenstangenhülse (3) und dem Federarm (5a) kann ein Klick-Geräusch erzeugen.

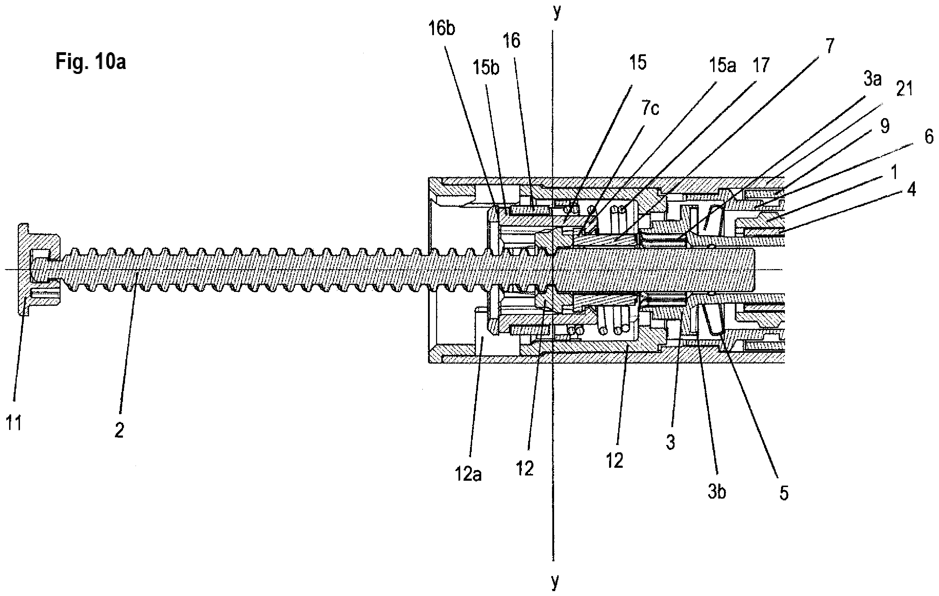

Figur 10a zeigt eine beispielhafte Dosiervorrichtung nach Ausschüttung der letzten Dosis, wobei die Dosiervorrichtung von dem Karpulenhalter losgelöst ist.

Der Karpulenhalter (18) weist eine Ausnehmung (18c) auf, welche mit der an einer Aussenmantelfläche vorgesehene Nocke (16c) des Kupplungshalters (16) in Eingriff sein kann, wie in den Figuren 1 und 2 ersichtlich ist. Bei Abnahme des Karpulenhalters (18), welcher mit einer Bajonettverbindung mit der Gewindehülse (12) verbunden ist, wird der Nocken (16c) des Kupplungshalters (16) mit dem Karpulenhalter (18) relativ zu der Gewindehülse (12) gedreht.

Die Kupplungsfeder (17) beaufschlagt den Karpulenhalter (18) mit einer in distale Richtung wirkende Federkraft. Der Kupplungsring (15) wird durch die Führungsnocke (15b), welche in Führungseingriff mit dem Karpulenhalter (16) ist in die distale Richtung bewegt. Da die Kupplungshülse (7) mittels drehbarer Schnappverbindung axial fest mit Kupplungsring (15) verbunden, wird die Kupplungshülse in distale Richtung bewegt, wobei die Rippen (7b) der Kupplungshülse (7) ausser Eingriff mit den Rillen (3a) der Kolbenstangenhülse (3) gelangen. Die drehfeste Verbindung zwischen der Kolbenstange (2) und der Kolbenstangenhülse (3) ist aufgelöst.

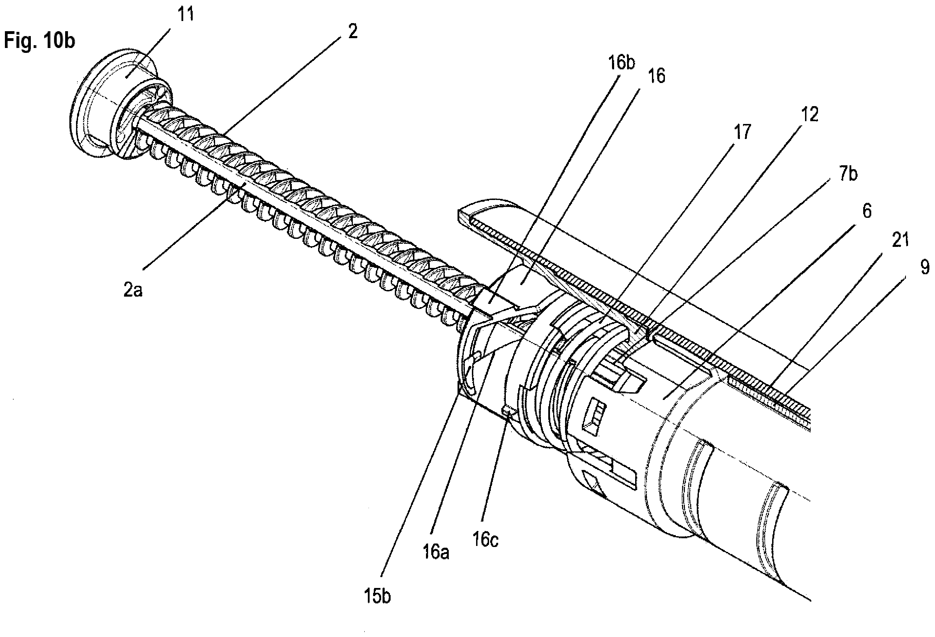

Figur 10b zeigt eine beispielhafte Dosiervorrichtung nach Ausschüttung der letzten Dosis in Perspektivansicht, wobei die Dosiervorrichtung von dem Karpulenhalter losgelöst ist.

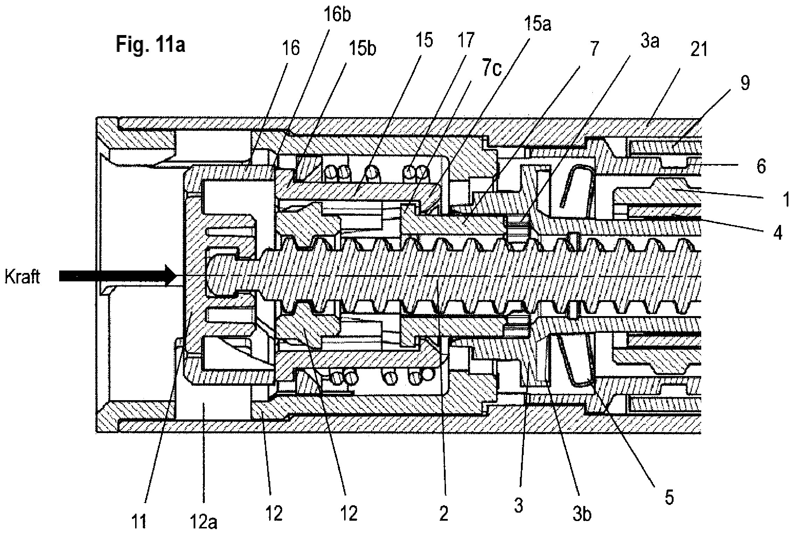

Figur 11a zeigt eine beispielhafte Dosiervorrichtung beim Einsetzen eines Karpulenhalters, wobei der Karpulenhalter nicht dargestellt ist. Die Kupplungsfeder (17) beaufschlagt den Kupplungshalter (16) mit einer in die distale Richtung wirkende Kraft. Aufgrund der Kupplung zwischen dem Kupplungshalter (16) dem Kupplungsring (15) und der Kupplungshülse (7) ist die Kupplungshülse (7) von der Kolbenstangenhülse (3) entkoppelt. Die Kolbenstange (3) ist somit drehbar zu der Kolbenstangenhülse (3) angeordnet. Um die Kolbenstange (2) zurück in die Dosiervorrichtung zu schieben, sodass die Dosiervorrichtung mit einer vollgefüllten Karpule (19) verbunden werden kann, wird eine axiale Kraft in proximale Richtung gegen den Flansch (11) ausgeübt. Bei dem Zusammenbringen des Karpulenhalters (18) mit der Dosiervorrichtung stösst insbesondere ein Kolben in der Karpule (19) gegen den Flansch, um die Kolbenstange in die Dosiervorrichtung zurückzuschieben. Die Kolbenstange (2) dreht sich in proximale Richtung in die Dosiervorrichtung ein, wobei sich die Kolbenstange (2) relativ zu dem Flansch (11) und zu der Gewindehülse (12) dreht. Der Karpulenhalter (18) wird zuerst axial in die Gewindehülse (12) eingeführt. Die Verschlussnocke (18a) des Karpulenhalters (18) führt den Karpulenhalter mittels Verschlussführung (12a) der Gewindehülse (12) in die Dosiervorrichtung ein. Die Ausnehmung (18c) des Karpulenhalters (18) nimmt die Nocke (16c) des Kupplungshalters (16) auf, um eine drehfeste Verbindung zu bilden. Der mit der Kupplungsfeder (17) beaufschlagte Kupplungshalter (16) stösst gegen die Karpule (19) an, um die Karpule (19) in den Karpulenhalter (18) axial zu fixieren. Durch eine relative Drehung des Karpulenhalters (18) zu der Gewindehülse (12) wird der Karpulenhalter (18) an die Dosiervorrichtung mittels Bajonettverbindung befestigt. Aufgrund der drehfesten Verbindung zwischen dem Karpulenhalter (18) und dem Kupplungshalter (16), wird der Kupplungshalter (16) ebenfalls relativ zu der Gewindehülse gedreht. Der Vorsprung (16b) des Karpulenhalters (16) gelangt mit der Ausnehmung der axialen Nut (nicht ersichtlich) der Gewindehülse (12) in Verrastung. Der zu der Gewindehülse drehfest und axial verschieblich angeordneter Kupplungsring wird aufgrund des Führungseingriffs zwischen der Führungsnocke (15b) des Kupplungsrings (15) und der Führungsbahn (16a) des Kupplungshalters (16) in die proximale Richtung bewegt. Die mit dem Kupplungsring (15) axial fest verbundene Kupplungshülse (7) wird ebenfalls in die proximale Richtung bewegt und gelangt in Eingriff mit der Kolbenstangenhülse (3). Die Rippenverbindung zwischen den Rippen (7b) der Kupplungshülse (7) und den Rillen (3a) der Kolbenstangenhülse ist wieder hergestellt. Die drehfeste Verbindung zwischen der Kolbenstange und der Kolbenstangenhülse ist wieder vorhanden.

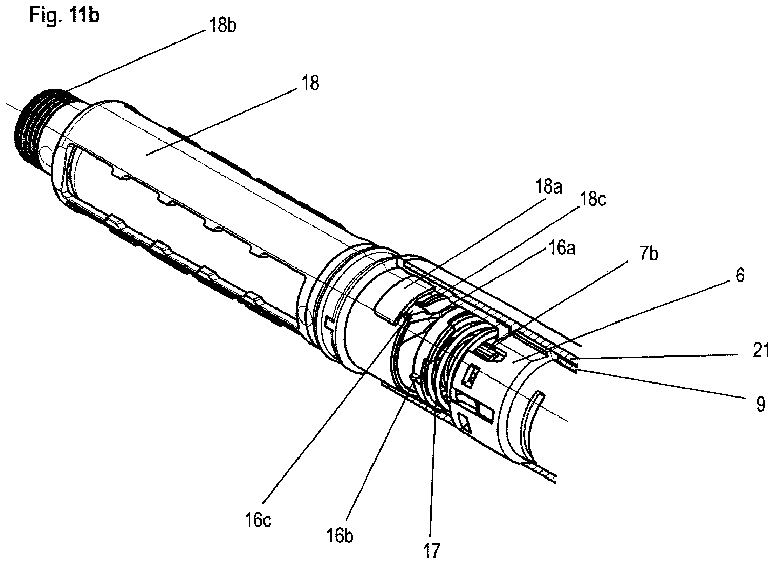

Figur 11b zeigt eine beispielhafte Dosiervorrichtung beim Einsetzen eines Karpulenthalters (18)

## Patentansprüche

1. Dosiervorrichtung für eine Injektionsvorrichtung mit
- einem Betätigungselement (1; 10; 100) zum Einstellen oder Abgeben einer Dosis aus der Injektionsvorrichtung,
- einer Kolbenstange (2) zum Erzeugen einer Vorschubbewegung zum Ausschütten einer Dosis,
- einer Kolbenstangenhülse (3), in welcher die Kolbenstange (2) derart aufgenommen ist, dass die Kolbenstange (2) mit der Kolbenstangenhülse (3) drehfest verbindbar ist,
- einem Kupplungselement (40), mit welchem das Betätigungselement (1; 10, 100) und die Kolbenstangenhülse (3) zum Abgeben der mit dem Betätigungselement (1; 10; 100) eingestellten Dosis derart gekoppelt werden kann, dass eine Ausschüttbewegung des Betätigungselements (1; 10; 100) über die mit der Kolbenstange (2) verbundenen Kolbenstangenhülse (3) auf die Kolbenstange (2) übertragen wird,
- wobei die Dosiervorrichtung ferner ein Rückdrehsicherungselement (5) für die Kolbenstangenhülse (3) aufweist, um eine Drehung der Kolbenstangenhülse (3) in eine Richtung zu ermöglichen und in Gegenrichtung zu sperren;
- **dadurch gekennzeichnet, dass** das Kupplungselement (40) ein Koppelglied (40a) und das Betätigungselement (10) ein Gegenkoppelglied (10c) aufweisen, welche durch eine radiale Verschiebung des Kupplungselements (40) relativ zu dem Betätigungselement (10) zueinander eingekoppelt oder geöffnet werden können.

2. Dosiervorrichtung nach Anspruch 1, **dadurch gekennzeichnet, dass** das Rückdrehsicherungselement (5) derart in Eingriff mit der Kolbenstangenhülse (3) ist, dass das Rückdrehsicherungselement (5) eine Drehung der Kolbenstangenhülse (5) in eine Richtung ermöglicht und in Gegenrichtung gesperrt.

3. Dosiervorrichtung nach Anspruch 1 oder 2, **dadurch gekennzeichnet, dass** das Rückdrehsicherungselement (5) und die Kolbenstangenhülse (3) gekoppelt sind.

4. Dosiervorrichtung nach einem der vorhergehenden Ansprüche, **dadurch gekennzeichnet, dass** das Rückdrehsicherungselement (5) und die Kolbenstangenhülse (3) mittels einer Verzahnung oder Verrastung in Eingriff sind.

5. Dosiervorrichtung nach einem der vorhergehenden Ansprüche, **dadurch gekennzeichnet, dass** das Rückdrehsicherungselement (5) zumindest teilweise spannbar oder elastisch deformierbar ausgebildet ist.

6. Dosiervorrichtung nach einem der vorhergehenden Ansprüche, **dadurch gekennzeichnet, dass** das Rückdrehsicherungselement (5) einen Federarm (5) aufweist oder als Federarm ausgebildet ist.

7. Dosiervorrichtung nach einem der vorhergehenden Ansprüche, **dadurch gekennzeichnet, dass** das Rückdrehsicherungselement (5) mit einem Gehäuse (6) der Dosiervorrichtung dreh- und axialfest verbunden ist.

8. Dosiervorrichtung nach einem der vorhergehenden Ansprüche, **dadurch gekennzeichnet, dass** das Kupplungselement (40) verdrehsicher mit der Kolbenstangenhülse (3) gekoppelt ist und verdrehsicher mit dem Betätigungselement (1) gekoppelt werden kann.

9. Dosiervorrichtung nach einem der vorhergehenden Ansprüche, **dadurch gekennzeichnet, dass** die Dosiervorrichtung ferner einen Dosierknopf (8; 80; 800) aufweist, welcher drehbar an dem Betätigungselement gelagert ist.

10. Dosiervorrichtung nach Anspruch 9, **dadurch gekennzeichnet, dass** der Dosierknopf (80) derart ausgebildet ist, dass er bei einer Vorschubbewegung auf das Kupplungselement (40) wirkt und eine radiale Verschiebung des Kupplungselements (40) relativ zu dem Betätigungselements (10) ermöglicht.

11. Dosiervorrichtung nach einem der vorhergehenden Ansprüche mit einer Gewindeführung für die ein Gewinde aufweisende Kolbenstange (2).

12. Injektionsvorrichtung mit einer Dosiervorrichtung nach einem der vorhergehenden Ansprüche.

13. Verfahren zum Einstellen und Abgeben einer Dosis aus einer Injektionsvorrichtung, wobei ein Betätigungselement (1; 10; 100) relativ zu der Injektionsvorrichtung und relativ zu einer Kolbenstange (2) zum Einstellen einer Dosis in eine Drehrichtung gedreht und zum Korrigieren einer Dosis in eine der Drehrichtung entgegengesetzten Richtung gedreht werden kann und wobei das Betätigungselement (1; 10; 100) über ein Kupplungselement (40) und eine Kolbenstangenhülse (3) mit der Kolbenstange (2) verdrehsicher gekoppelt wird, wenn die Dosis aus der Injektionsvorrichtung ausgeschüttet wird, wobei eine Rückdrehsicherungselement (5) für die Kolbenstangenhülse (3) eine Drehung der Kolbenstangenhülse (3) in eine Richtung ermöglicht und in Gegenrichtung sperrt, **dadurch gekennzeichnet, dass** das Kupplungselement (40) ein Koppelglied (40a) und das Betätigungselement (10) ein Gegenkoppelglied (10c) aufweisen, welche durch eine radiale Verschiebung des Kupplungselements (40) relativ zu dem Betätigungselement (10) zueinander eingekoppelt oder geöffnet werden können.

14. Verfahren nach Anspruch 13, **dadurch gekennzeichnet, dass** das Rückdrehsicherungselement (5) in die Kolbenstangenhülse (3) eingreift, um eine Drehung der Kolbenstangenhülse (3) in eine Richtung zu ermöglichen und in Gegenrichtung zu sperren.

15. Verfahren nach Anspruch 14, **dadurch gekennzeichnet, dass** das Rückdrehsicherungselement (5) federnd oder elastische deformierbar in die Kolbenstangenhülse (3) eingreift, um eine Drehung der Kolbenstangenhülse (3) in eine Richtung zu ermöglichen und in die Gegenrichtung zu sperren, wobei bei der Drehung der Kolbenstangenhülse (3) relativ zu dem Rückdrehsicherungselement (5) ein Klick-Geräusch erzeugt wird.

16. Verfahren nach einem der Ansprüche 13 bis 15, **dadurch gekennzeichnet, dass** das Drehmoment zwischen dem Rückdrehsicherungselement (5) und der Kolbenstangenhülse (3) grösser als das Drehmoment zwischen dem Betätigungselement (1; 10; 100) und dem Kupplungselement (40) ist.

## Claims

1. A metering apparatus for an injection device, comprising
- an actuating element (1; 10; 100) for setting or discharging a dose from the injection device,
- a piston rod (2) for producing an advance movement for dispensing a dose,
- a piston rod sleeve (3) in which the piston rod (2) is so accommodated that the piston rod (2) can be non-rotatably connected to the piston rod sleeve (3),
- a coupling element (4; 40; 400) with which the actuating element (1; 10; 100) and the piston rod sleeve (3) can be coupled for discharge of the dose set with the actuating element (1; 10; 100) in such a way that a dispensing movement of the actuating element (1; 10; 100) is transmitted to the piston rod (2) by way of the piston rod sleeve (3) connected to the piston rod (2),
- wherein the metering apparatus further has a reverse rotation prevention element (5) for the piston rod sleeve (3), to permit rotation of the piston rod sleeve (3) in one direction and to block it in the opposite direction,
- **characterised in that** the coupling element (40) has a coupling member (40a) and the actuating element (10) has a counterpart coupling member (10c) which can be coupled or opened relative to each other by radial displacement of the coupling element (40) relative to the actuating element (10).

2. A metering apparatus according to claim 1 **characterised in that** the reverse rotation prevention element (5) is in engagement with the piston rod sleeve (3) in such a way that the reverse rotation prevention element (5) permits rotation of the piston rod sleeve (5) in one direction and blocks it in the opposite direction.

3. A metering apparatus according to claim 1 or claim 2 **characterised in that** the reverse rotation prevention element (5) and the piston rod sleeve (3) are coupled.

4. A metering apparatus according to one of the preceding claims **characterised in that** the reverse rotation prevention element (5) and the piston rod sleeve (3) are in engagement by means of a tooth means or a latching means.

5. A metering apparatus according to one of the preceding claims **characterised in that** the reverse rotation prevention element (5) is adapted to be at least partially tightenable or elastically deformable.

6. A metering apparatus according to one of the preceding claims **characterised in that** the reverse rotation prevention element (5) has a spring arm (5) or is in the form of a spring arm.

7. A metering apparatus according to one of the preceding claims **characterised in that** the reverse rotation prevention element (5) is non-rotatably and axially fixedly connected to a housing (6) of the metering apparatus.

8. A metering apparatus according to one of the preceding claims **characterised in that** the coupling element (4; 40; 400) is non-rotatably coupled to the piston rod sleeve (3) and can be non-rotatably coupled to the actuating element (1).

9. A metering apparatus according to one of the preceding claims **characterised in that** the metering apparatus further has a metering knob (8; 80; 800) mounted rotatably to the actuating element.

10. A metering apparatus according to claim 9 **characterised in that** the metering knob (80) is so designed that upon an advance movement it acts on the coupling element (40) and permits a radial displacement of the coupling element (4; 40; 400) relative to the actuating element (10).

11. A metering apparatus according to one of the preceding claims having a thread guide means for the piston rod (2) having a thread.

12. An injection device having a metering apparatus according to one of the preceding claims.

13. A method of setting and discharging a dose from an injection device, wherein an actuating element (1; 10; 100) can be rotated in one direction of rotation relative to the injection device and relative to a piston rod (2) for setting a dose and can be rotated in a direction opposite to the direction of rotation for correcting a dose and wherein the actuating element (1; 10; 100) is non-rotatably coupled to the piston rod (2) by way of a coupling element (4; 40; 400) and a piston rod sleeve (3) when the dose is dispensed from the injection device, wherein a reverse rotation prevention element (5) for the piston rod (2) permits rotation of the piston rod sleeve (3) in one direction and blocks it in the opposite direction,
**characterised in that** the coupling element (40) has a coupling member (40a) and the actuating element (10) has a counterpart coupling member (10c) which can be coupled or opened relative to each other by radial displacement of the coupling element (40) relative to the actuating element (10).

14. A method according to claim 13 **characterised in that** the reverse rotation prevention element (5) engages into the piston rod sleeve (3) to permit rotation of the piston rod sleeve (3) in one direction and to block it in the opposite direction.

15. A method according to claim 14 **characterised in that** the reverse rotation prevention element (5) engages resiliently or elastically deformably into the piston rod sleeve (3) to permit rotation of the piston rod sleeve (3) in one direction and to block it in the opposite direction, wherein upon rotation of the piston rod sleeve (3) relative to the reverse rotation prevention element (5) a clicking sound is produced.

16. A method according to one of claims 13 to 15 **characterised in that** the moment of rotation between the reverse rotation prevention element (5) and the piston rod sleeve (3) is greater than the moment of rotation between the actuating element (1; 10; 100) and the coupling element (4; 40; 400).

## Revendications

1. Dispositif de dosage pour un dispositif d'injection avec
- un élément d'actionnement (1 ; 10 ; 100) pour le réglage ou la distribution d'une dose du dispositif d'injection,
- une tige de piston (2) pour la génération d'un mouvement d'avancement pour le déversement d'une dose,
- une douille de tige de piston (3), dans laquelle la tige de piston (2) est reçue de sorte que la tige de piston (2) puisse être reliée solidaire en rotation à la douille de tige de piston (3),
- un élément d'accouplement (40), avec lequel l'élément d'actionnement (1 ; 10, 100) et la douille de tige de piston (3) peuvent être couplés pour la distribution de la dose réglée avec l'élément d'actionnement (1 ; 10 ; 100) de sorte qu'un mouvement de déversement de l'élément d'actionnement (1 ; 10 ; 100) soit transmis sur la tige de piston (2) par le biais de la douille de tige de piston (3) reliée à la tige de piston (2),
- dans lequel le dispositif de dosage présente en outre un élément anti-rotation arrière (5) pour la douille de tige de piston (3) pour permettre une rotation de la douille de tige de piston (3) dans un sens et la bloquer dans le sens opposé ;
- **caractérisé en ce que** l'élément d'accouplement (40) présente un organe de couplage (40a) et l'élément d'actionnement (10), un organe de contre-couplage (10c), lesquels peuvent être couplés ou ouverts l'un par rapport à l'autre par un déplacement radial de l'élément d'accouplement (40) par rapport à l'élément d'actionnement (10).

2. Dispositif de dosage selon la revendication 1, **caractérisé en ce que** l'élément anti-rotation arrière (5) est en prise avec la douille de tige de piston (3) de sorte que l'élément anti-rotation arrière (5) permette une rotation de la douille de tige de piston (5) dans un sens et la bloque dans le sens opposé.

3. Dispositif de dosage selon la revendication 1 ou 2, **caractérisé en ce que** l'élément anti-rotation arrière (5) et la douille de tige de piston (3) sont couplés.

4. Dispositif de dosage selon l'une quelconque des revendications précédentes, **caractérisé en ce que** l'élément anti-rotation arrière (5) et la douille de tige de piston (3) sont en prise au moyen d'une denture ou enclenchement.

5. Dispositif de dosage selon l'une quelconque des revendications précédentes, **caractérisé en ce que** l'élément anti-rotation arrière (5) est réalisé au moins partiellement apte à être serré ou élastiquement déformable.

6. Dispositif de dosage selon l'une quelconque des revendications précédentes, **caractérisé en ce que** l'élément anti-rotation arrière (5) présente un bras ressort (5) ou est réalisé en tant que bras ressort.

7. Dispositif de dosage selon l'une quelconque des revendications précédentes, **caractérisé en ce que** l'élément anti-rotation arrière (5) est relié solidaire en rotation et axialement à un boîtier (6) du dispositif de dosage.

8. Dispositif de dosage selon l'une quelconque des revendications précédentes, **caractérisé en ce que** l'élément d'accouplement (40) est couplé bloqué en rotation avec la douille de tige de piston (3) et peut être couplé bloqué en rotation avec l'élément d'actionnement (1).

9. Dispositif de dosage selon l'une quelconque des revendications précédentes, **caractérisé en ce que** le dispositif de dosage présente en outre un bouton de dosage (8 ; 80 ; 800), lequel est logé en rotation au niveau de l'élément d'actionnement.

10. Dispositif de dosage selon la revendication 9, **caractérisé en ce que** le bouton de dosage (80) est réalisé de sorte qu'il agisse lors d'un mouvement d'avancement sur l'élément d'accouplement (40) et permette un déplacement radial de l'élément d'accouplement (40) par rapport à l'élément d'actionnement (10).

11. Dispositif de dosage selon l'une quelconque des revendications précédentes avec un guidage fileté pour la tige de piston (2) présentant un filetage.

12. Dispositif d'injection avec un dispositif de dosage selon l'une quelconque des revendications précédentes.

13. Procédé de réglage et de distribution d'une dose d'un dispositif d'injection, dans lequel un élément d'actionnement (1 ; 10 ; 100) est tourné dans un sens de rotation par rapport au dispositif d'injection et par rapport à une tige de piston (2) pour le réglage d'une dose et peut être tourné dans un sens opposé au sens de rotation pour la correction d'une dose et dans lequel l'élément d'actionnement (1 ; 10 ; 100) est couplé bloqué en rotation avec la tige de piston (2) par le biais d'un élément d'accouplement (40) et d'une douille de tige de piston (3), lorsque la dose est déversée du dispositif d'injection, dans lequel un élément anti-rotation arrière (5) pour la douille de tige de piston (3) permet une rotation de la douille de tige de piston (3) dans un sens et la bloque dans le sens opposé, **caractérisé en ce que** l'élément d'accouplement (40) présente un organe de couplage (40a) et l'élément d'actionnement (10), un organe de contre-couplage (10c), lesquels peuvent être couplés ou ouverts l'un par rapport à l'autre par un déplacement radial de l'élément d'accouplement (40) par rapport à l'élément d'actionnement (10).

14. Procédé selon la revendication 13, **caractérisé en ce que** l'élément anti-rotation arrière (5) vient en prise avec la douille de tige de piston (3) pour permettre une rotation de la douille de tige de piston (3) dans un sens et la bloquer dans le sens opposé.

15. Procédé selon la revendication 14, **caractérisé en ce que** l'élément anti-rotation arrière (5) vient en prise élastiquement ou élastiquement déformable avec la douille de tige de piston (3) pour permettre une rotation de la douille de tige de piston (3) dans un sens et la bloquer dans le sens opposé, dans lequel lors de la rotation de la douille de tige de piston (3) par rapport à l'élément anti-rotation arrière (5), un bruit de clic est généré.

16. Procédé selon l'une quelconque des revendications 13 à 15, **caractérisé en ce que** le couple entre l'élément anti-rotation arrière (5) et la douille de tige de piston (3) est supérieur au couple entre l'élément d'actionnement (1 ; 10 ; 100) et l'élément d'accouplement (40).
